# EUROPÄISCHE PATENTSCHRIFT

(11) **EP 0 581 250 B1**
(45) Veröffentlichungstag und Bekanntmachung des Hinweises auf die Patenterteilung: **03.03.1999**
(21) Anmeldenummer: 93112006.7
(22) Anmeldetag: 27.07.1993
(51) Int. Cl.: C07D 333/24, C07D 409/06, C07D 333/28, C07D 333/42, C12P 17/00, C07K 1/00

(54) **Verfahren zur Biotechnischen Herstellung von L-Thienylalaninen in enantiomerenreiner Form aus 2-Hydroxy-3-thienyl-acrylsäuren und ihre Verwendung**
Process for biotechnical preparation of L-thienylalanines in enantiomere pure form from 2-hydroxy-3-thienyl-acrylic acids and their use
Procédé de préparation biotechnique de L-thiénylalanines sous forme d'énantiomères pure à partir d'acides 2-hydroxy-3-thiényl-acryliques et leur application

(30) Priorität: 31.07.1992 DE 4225280
(43) Veröffentlichungstag der Anmeldung: 02.02.1994
(73) Patentinhaber: HOECHST AKTIENGESELLSCHAFT, 65929 Frankfurt am Main (DE)
(72) Erfinder: Kretzschmar, Gerhard, Dr., D-65760 Eschborn (DE); Meiwes, Johannes, Dr., D-65510 Idstein (DE); Schudok, Manfred, Dr., D-65795 Hattersheim/Main (DE); Hammann, Peter, Dr., D-64832 Babenhausen (DE); Lerch, Ulrich, Dr., D-65719 Hofheim am Taunus (DE); Grabley, Susanne, Dr., D-61462 Königstein/Taunus (DE)

(56) Entgegenhaltungen:
- EP-A- 0 152 275
- EP-A- 0 189 938
- EP-A- 0 248 357
- WO-A-89/12688
- CH-A- 676 846
- LIEBIGS ANNALEN DER CHEMIE. Bd. 703 , 1967 , WEINHEIM DE Seiten 37 - 43 L. HORNER ET AL. 'alpha-Methoxy, alpha-Äthylmercapto- und alpha-Dimethylamino-acrylester aus substituierten Essigestern mit Aldehyden'
- JOURNAL OF THE CHEMICAL SOCIETY, PERKIN TRANSACTIONS 1. Nr. 4 , April 1990 , LETCHWORTH GB Seiten 1151 - 1158 CH. M. BLADON 'Synthesis of Heteroaromatic Thyrotropin-releasing Hormone Analogues'
- CHEMICAL REVIEWS Bd. 83, Nr. 1-6 , 1983 Seiten 321 - 358 A.J.L. COOPER ET AL. 'Synthesis and Properties of the alpha-Keto Acids'

## Beschreibung

Die Herstellung von L-Thienylalaninen erfolgt über die Hydantoin- oder die Azlacton-Route. Als Ausgangssubstanz für die Biotransformation dienen 2-Hydroxy-3-thienylacrylsäuren. Der innovative Schritt besteht in der Transaminierung der Enolform der 2-Hydroxy-3-thienylacrylsäuren zu L-Thienylalaninen mit Hilfe der Biotransformation. Die Transaminierung erfolgt in Gegenwart von L-Asparagin- oder L-Glutaminsäure als Aminodonor.

In der CH 6 76 846 ist ein Verfahren zur Herstellung von C-5-ethylenisch ungesättigten Hydantoinderivaten als Zwischenprodukte für die Herstellung von z.B. Phenylalanin und Phenylbrenztraubensäure beschrieben, wobei z.B. ein aromatischer Aldehyd mit einem Dialkyl-5-phosphonat-hydantoin umgesetzt wird.

Optisch aktive, nicht-proteinogene Aminosäuren, wie z. B. L-Thienylalanin, haben große Bedeutung als Pharmaka, Pflanzenschutzmittel oder Synthesebausteine. Beispiele sind L-Dopa gegen die Parkinson'sche Krankheit, alpha-Methyldopa gegen Bluthochdruck oder L-Phosphinothricin als biologisch aktive Komponente eines Herbizidwirkstoffes.

Außerdem sind optisch aktive Aminosäuren Synthesevorstufen, insbesondere für Pharmazeutika, wie z. B. D-Phenylglycin oder D-Parahydroxyphenylglycin bei der Herstellung semisynthetischer Penicilline. Sie werden außerdem als chirale Synthesebausteine für andere chirale Feinchemikalien sowie für den Einbau in modifizierte biologisch wirksame Peptide verwendet [R. A. Sheldon, H. J. M. Zeegers, J. P. M. Houbiers und L. A. Hulshof, Chimicaoggi, 5, S. 35 (1991)].

Die nicht-proteinogenen optisch aktiven Aminosäuren sind bisher - weil nicht fermentativ oder aus natürlichen Quellen gewinnbar - durch konventionelle Synthese mit anschließender Racematspaltung, durch asymmetrische Synthese mit chiralen Hilfsstoffen oder durch Biotransformation chiraler oder prochiraler Vorstufen hergestellt worden.

Zur kommerziellen Synthese von nicht-proteinogenen Aminosäuren werden z. B. folgende Verfahren angewandt:
1. Das Amidase-Verfahren, bei dem racemische Aminosäureamide durch Hydrolyse mit einer L-spezifischen Aminopeptidase gespalten werden [W. H. J. Boesten, US-Patent 3,971,700 (1976)].
2. Die Acylase-katalysierte enantioselektive Hydrolyse der N-Acetyl-D,L-Aminosäuren [I. Chibata und T. Tosa, Ann. Rev. Biophys. Bioengin., 10, 197 (1981)].

Beide Verfahren besitzen den Nachteil der nur maximal 50 %igen Ausbeute des optisch aktiven Reaktionsprodukts.

Bei einem fortgeschritteneren biotechnologischen Verfahren, der mikrobiellen Hydrolyse D,L-monosubstituierter Hydantoine, kann das chirale Vorprodukt der Aminosäure durch die Gegenwart einer Racemase mit über 50 %iger Ausbeute in die enantiomerenreine L-Aminosäure überführt werden [Ch. Syldatk, A. Läufer, R. Müller und H. Höke in Advances Biochem. Engin. in Biotechnol. Vol. 41, A. Fiechter (Ed.), S. 29 bis 75 Vlg. Springer, Berlin, New York, London (1990)]. Die Nachteile dieses Verfahrens liegen zum einen in der schwierigen Synthese der gesättigten (hydrierten) Hydantoin-Vorstufen, insbesondere wenn schwefelhaltige Teilstrukturen enthalten sind, die zu einer Inaktivierung von Edelmetallhydrierkatalysatoren führen und aufwendigere elektrochemische Reduktionsverfahren zur Bereitstellung der Vorstufen erforderlich machen (H. Hoeke, Conference Paper, Chiral '92, Manchester UK 1992). Zum anderen ist dieses Verfahren technisch sehr aufwendig, da es ein vielschrittiger Prozess ist, bei welchem mindestens drei Enzyme eingesetzt werden. Die unterschiedlichen Aktivitäten der beteiligten Enzyme bei bestimmten pH-Werten und Temperaturen erfordern eine anspruchsvolle Verfahrensführung [A. Möller, C.Syldatk, M.Schulze, F.Wagner, Enzyme Microbiol. Technol. 10, S. 618, (1988)]

Eine weitere Möglichkeit der enantiospezifischen Synthese von Aminosäuren besteht in der Transaminierung prochiraler alpha-Ketosäure-Vorstufen. Dieses Verfahren wurde vor allem zur Synthese natürlicher Aminosäuren, aber auch für nicht-proteinogene Aminosäuren beschrieben [Synthese von L-Leucin aus alpha-Ketoisocapronsäure mit einem Corynebacterium glutamicum-Stamm (R. Wichmann, C. Wandrey und I. Große-Weismann, J. Appl. Microbiol. Biotechnol., 32, S. 373 (1990)).

In der Europäischen Patentanmeldung 0 152 275 wird ein Verfahren zur Herstellung der proteinogenen Aminosäure Phenylalanin mit Hilfe eines genetisch modifizierten Mikroorganismus beschrieben, der sich durch Überproduktion der Aminotransferase auszeichnet.

Auch nicht-proteinogene Aminosäuren werden durch mikrobielle oder enzymatische Biotransformation der entsprechenden alpha-Ketosäuren gewonnen. Beispiele hierfür sind das Verfahren zur Herstellung von L-tertiär-Leucin und L-Phosphinothricin mit einem genetisch modifizierten E.coli Stamm (EP 0 248 357) sowie die Transaminierung einer Reihe von alpha-Ketosäuren mit einer isolierten Aspartat-Aminotransferase aus E.coli und Glutaminsäure als Amino-Donor [J. E. Baldwin und S. C. Ng, Bull. Sing. N. I. Chem., 18, S. 127 (1990)].

Für die Synthese der optisch aktiven, nicht-proteinogenen Thienylalanine wurden bisher folgende, auf den Grundstrukturtyp des L-3-(2-Thienyl)-alanins beschränkte Verfahren beschrieben:
a) Die klassische Racematspaltung durch Kristallisation diastereomerer Salze [A. W. Lipkowski und G. Flouret, Pol. J. Chem., 54, S. 2225 (1980)];
b) Die enantioselektive Hydrolyse niederer Alkylester mittels alpha-Chymotrypsin [M. Pugniere, L. G. Barry und A. Previero, Biotechnol. Techniques, 3, S. 339 (1989)];
c) Die Racematspaltung des entsprechenden Aminosäureamids mit einer Aminopeptidase nach dem Verfahren von R.A.Sheldon (in "Chiral Synthesis", Proceedings of the Chiral Synthesis Workshop, Manchester, U.K., 18th April 1989, Conference Paper, Seite 25);
d) Das Hydantoinase-Verfahren von Ch. Syldatk et. al. [Ch. Syldatk, A. Läufer, R. Müller und H. Höke in Advances Biochem. Engin. in Biotechnol. Vol. 41, A. Fiechter (Ed.), S. 29 bis 75, Vlg. Springer, Berlin, New York, London (1990)] sowie
e) Die enzymatische Addition von Ammoniak an trans-3-(2-Thienyl)-acrylsäure (WO 8912-688).

Die unter a) bis c) genannten Verfahren besitzen den schon erwähnten Ausbeutenachteil aller racemischer Verfahren.
Während der Nachteil des Hydantoinase-Verfahrens zur Synthese von Thienylalanin vor allem in der schwierigen Zugänglichkeit des benötigten 5-Thienylmethylhydantoins liegt, arbeitet das zuletzt genannte Verfahren in hoher Substratverdünnung von nur ca. 3 g/l und liefert im 100 Milligramm-Maßstab die gewünschte Aminosäure L-3-(2-Thienyl)-alanin in 47,9 mg (43%) Ausbeute. Das Verfahren ist für Synthesen im technischen Maßstab nicht geeignet.

Es wurde nun überraschend ein Verfahren zur Herstellung von L-Thienylalaninen in enantiomerenreiner Form aus 2-Hydroxy-3-thienylacrylsäuren gefunden, wobei die Enolform der 2-Hydroxy-3-thienylacrylsäuren in Gegenwart von L-Asparaginsäure oder L-Glutaminsäure als Aminodonor in die jeweilige nicht-proteinogene Aminosäure transaminiert wird.

Die Erfindung betrifft somit
1. Ein Verfahren zur Herstellung von Thienylalaninen der Formel I 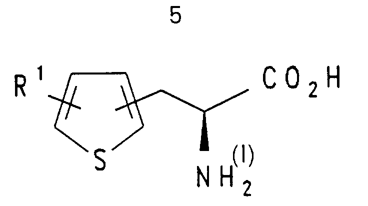 in welcher R¹ Wasserstoff, einen Halogen-, Nitro-, einen geradkettigen oder verzweigten Alkylrest bedeutet und der 2-Hydroxyacrylsäurerest in der 2- oder 3-Stellung des Thiophenrings steht bzw. die Salze dieser Verbindungen, das dadurch gekennzeichnet ist, daß die Verbindungen der Formel II 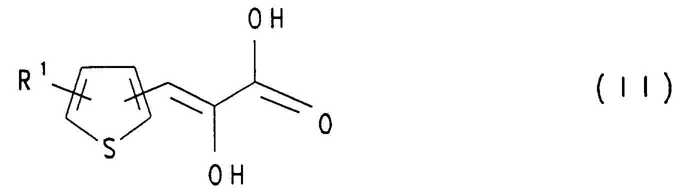 in welcher R¹ die obengenannten Bedeutungen hat, in Gegenwart der Aminosäuren Asparagin, Glutamin, L-Asparaginsäure und/oder L-Glutaminsäure und in Anwesenheit von Mikroorganismen oder Enzymen mit Transaminaseaktivität biotransformiert werden.
2. Die Verwendung der Verbindungen der Formel I als Baustein für die Peptidsynthese.

Im folgenden wird die Erfindung detailliert beschrieben und in den Patentansprüchen definiert.

Als Thienylalanine werden nicht-proteinogene Aminosäuren bezeichnet, die aus einem substituierten oder nicht-substituierten Thiophengrundkörper bestehen, der in 2'- oder 3'-Position an die Alaninseitenkette geknüpft ist.

Das Verfahren zur Synthese von L-Thienylalaninen kann im Labormaßstab (Herstellung von Mengen < 100 g) wie auch im industriellen Maßstab durchgeführt werden.

Darüber hinaus kann das Verfahren kontinuierlich oder "batchweise" durchgeführt werden. Kontinuierliches Verfahren in einem chemischen Reaktionsapparat - auch Fließbetrieb genannt - bedeutet für den Fachmann eine ununterbrochene Zufuhr der Reaktionspartner und ununterbrochene Abfuhr der Reaktionsprodukte.

Unter einem Verfahren, das "batchweise" - auch "Satzbetrieb" genannt - durchgeführt wird, versteht der Fachmann eine Reaktionsdurchführung, bei welcher eine stufenweise Hinzugabe oder Abnahme von Reaktionsmaterialen erfolgen kann.

Als industrieller Maßstab wird die Synthese von L-Thienylalaninen in Mengen ab 100 g definiert.

Die als Ausgangssubstanz verwendeten Thienylaldehyde der Formel I, beispielsweise 4-Br, 5-Br, 3-Methyl-, 5-Methyl-, 5-Nitro-Thiophenaldehyde, sind entweder käuflich oder nach dem Fachmann allgemein geläufigen Syntheseverfahren aus käuflichen Vorstufen herstellbar.

Die Herstellung von L-Thienylalaninen kann über folgende Reaktionsschritte erfolgen, die mit Ausnahme der Biotransformation der jeweiligen Enolform zum Stand der Technik gehören:
a) die Hydantoin-Route (Schema 1) oder
b) die Azlacton-Route (Schema 2).

Der in Schema 1 dargestellte Syntheseweg wird in Schema 3 anhand der Synthese des L-3-(2-Thienyl)-alanins (2A) näher erläutert.

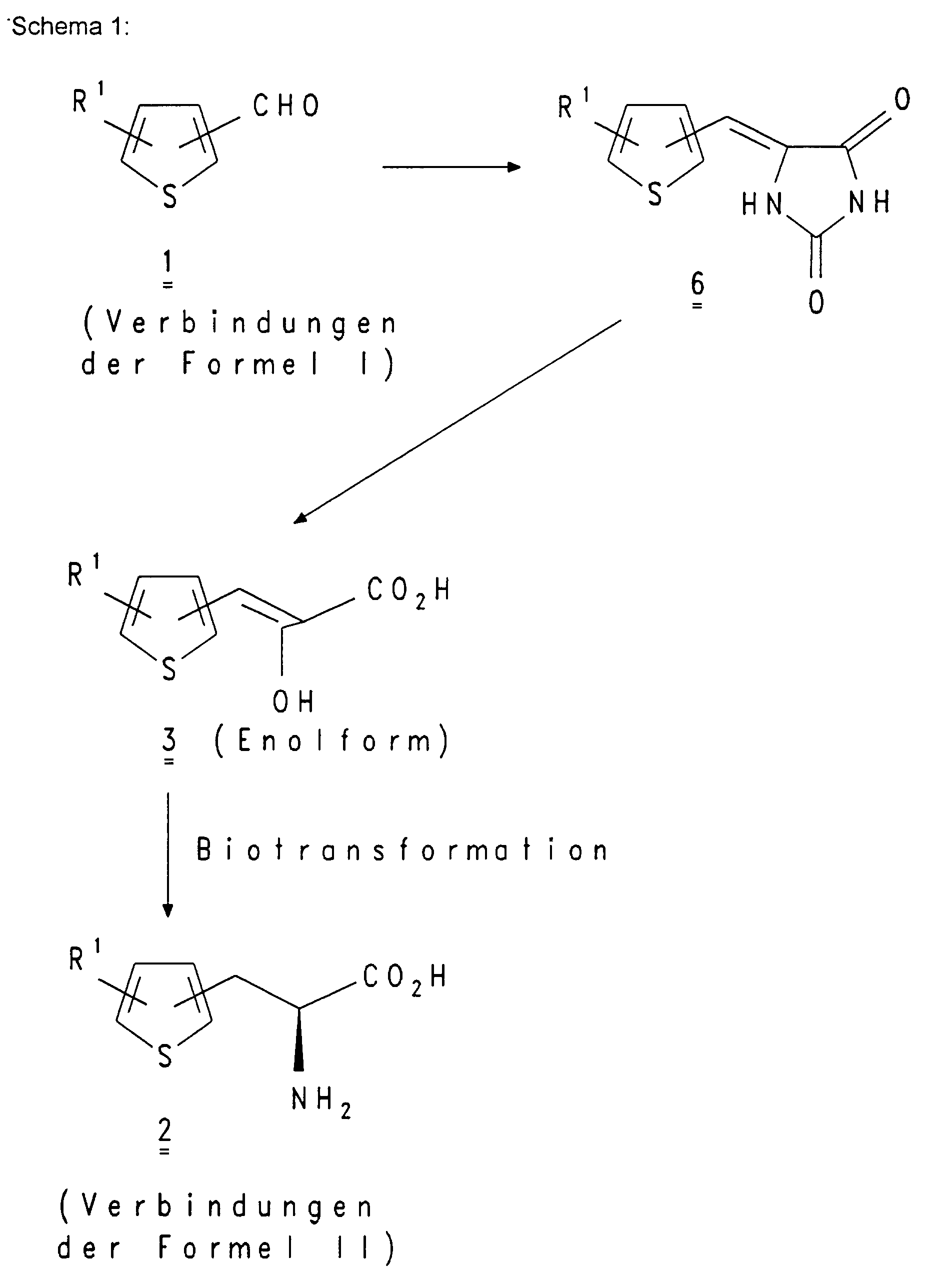

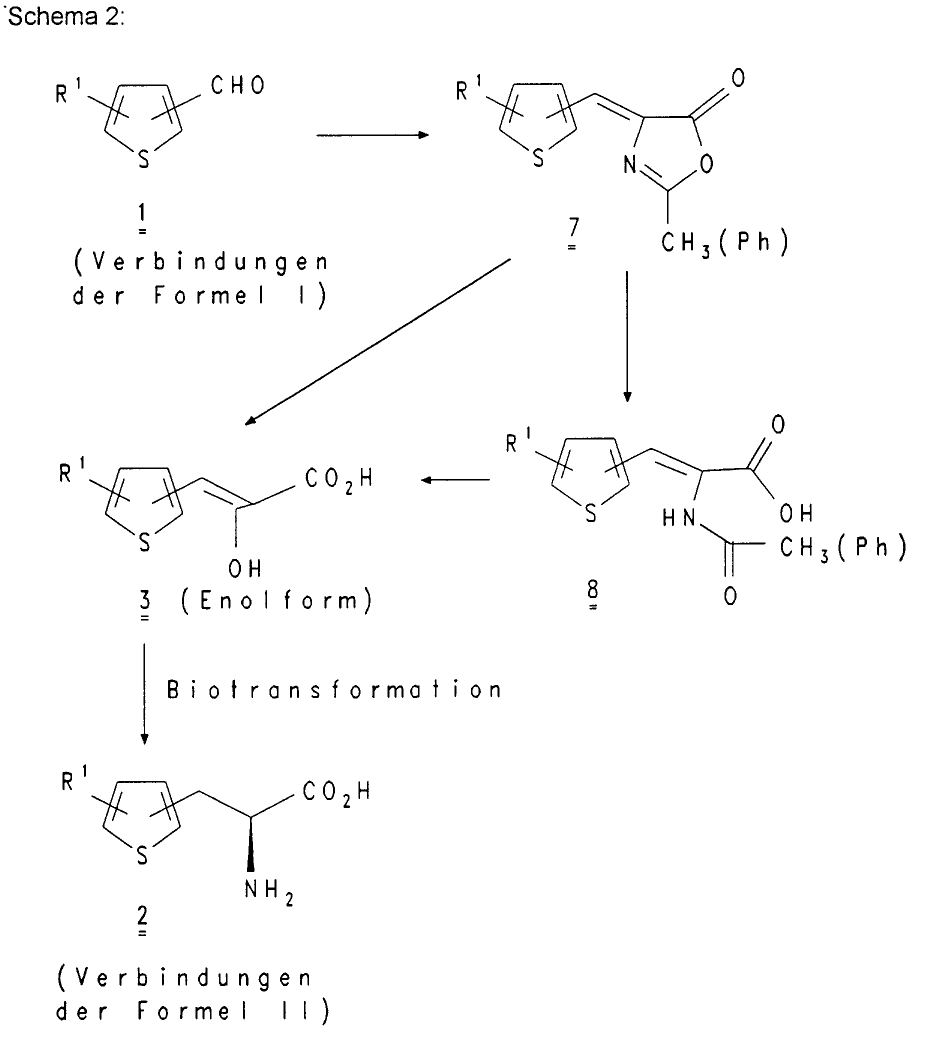

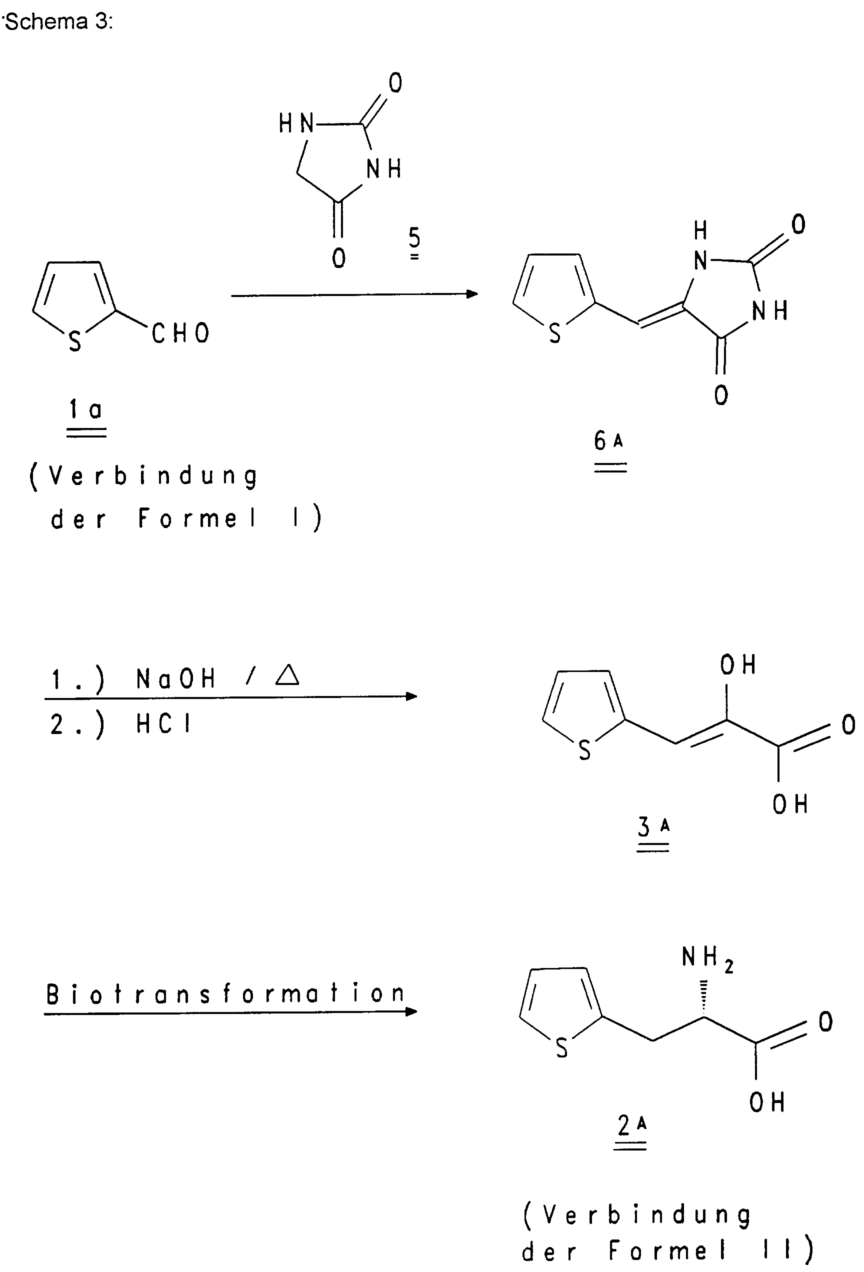

Wie in den Schemata gezeigt, werden die 2-Hydroxy-3-thienyl-acrylsäuren (3) durch hydrolytische Ringöffnung der entsprechenden heterocyclischen Vorstufen (6) bzw. (7) gewonnen. Die Hydantoinderivate (6) sind nach literaturbekannten Verfahren, z. B. durch Aldolkondensation mit unsubstituiertem Hydantoin (5), aus den Aminosäuren mit Metallcyanaten oder aus den 3-Thienyl-substituierten Propanalen mit Metallcyaniden und Harnstoff nach der Bucherer-Berg-Synthese erhältlich (E. Ware, "The Chemistry of Hydantoins", in Chem. Rev., R. L. Shriner (Ed.), Vol. 46, 403 - 470, The Williams& Wilkins Comp., Baltimore (1950)). Die Synthese der Enolcarbonsäuren (3) aus den Hydantoinderivaten (6) wurde noch nicht in der Literatur beschrieben und verläuft überraschenderweise in hohen Ausbeuten durch einfaches Erhitzen der Hydantoine in wäßrigen Basen bis zum Siedepunkt der wäßrigen Lösung.

Die Synthese der Enolcarbonsäuren (3; siehe Schema 2)) aus den Azlactonderivaten (7) kann grundsätzlich in enger Analogie zu den literaturbekannten Verfahren durch Erhitzen in wäßrigen Säuren oder Basen durchgeführt werden (J. P. Greenstein und M. Winitz, "Chemistry of the Amino Acids", Wiley, New York 1961). Die Hydrolyse der Azlactone (7) kann auch in zwei Schritten (Schema 2) über die 2-Benzoylamido- oder 2-Acetylamido-acrylate (8) als Zwischenstufen durchgeführt werden (B. F. Crowe und F. F. Nord, J. Org. Chem. 15, 1177 (1950)).

Eine Reihe von weiteren Synthesen für alpha-Ketocarbonsäuren, die grundsätzlich ebenfalls zur Herstellung der mit Formel (3) bezeichneten Zwischenprodukte herangezogen werden können, finden sich in dem Übersichtsartikel von A.J.L. Cooper, J.Z. Ginos, A. Meister, Chem. Rev. 83, 321 (1983). Die in den Schemata 1 - 3 aufgeführten Synthesewege sind jedoch hinsichtlich Ausbeuten, guter Zugänglichkeit der benötigten Chemikalien und aus Kostengründen für die technische Realisierung zu bevorzugen.

Die auf verschiedenen Synthesewegen hergestellten Vorstufen in der Enolform (3), lassen sich durch das erfindungsgemäße Verfahren im Sinne einer Transaminierungsreaktion in die Aminosäuren (2) umsetzen. Die spektroskopischen Befunde (Infrarot-Spektroskopie, Kernresonanzspektroskopie) belegen innerhalb der Nachweisgrenzen dieser Analysentechniken, daß in organischen und wäßrigen Lösemitteln sowie in weiten pH-Bereichen (pH 1-14), insbesondere unter den pH-Bedingungen der Biotransformation, ausschließlich die tautomeren Verbindungen (3) mit der in der Schemata 1-3 gezeichneten Enolstruktur vorliegen. Dieser Befund steht im Gegensatz zu Angaben in der Literatur, beispielsweise für die Verbindung 3A (2-Thienylrest und R¹=H), die in der Literatur mit der entsprechenden Ketosäurestrukturformel (4A) beschrieben wurde (L.Horner und E.O-Renth, Liebigs Ann. Chem., 703,37 (1967). Dagegen sind die entsprechenden Enolstrukturen von Estern der betreffenden 2-Hydroxy-3-Thienylacrylsäuren und der entsprechenden 2-Mercaptoverbindungen in der Literatur als stabile Tautomerieformen beschrieben worden (2-Thienylthiobrenztraubensäure in: B.F. Crowe und F.F. Nord, J. Org. Chem. 15,81 (1950); Ester in: A.M. Stock, W.E. Donahue und E.D. Amstutz, J. Org. Chem. 23, 1840 (1958)). Arylsubstituierte beta-Brenztraubensäuren der allgemeinen Formel Aryl-CH(2)-C(=O)CO(2)H liegen allgemein nur in stark basischem Milieu in bedeutendem Umfang in der tautomeren Enolform vor. Der Enolgehalt unter physiologischen pH-Bedingungen ist sehr niedrig (A.J.L. Cooper, J.Z. Ginos und A. Meister, Chem. Rev. 83,321 (1983)). So liegt beispielweise das Natriumsalz der Phenylbrenztraubensäure laut NMR-Spektrum fast ausschließlich in der Ketosäureform vor (The Aldrich Library of NMR-Spectra, 2. Auflage, Vol.2, 143C, S.2).

Zur Biotransformation der Enolcarbonsäuren (3) zu den Aminosäuren (2) können erfindungsgemäß alle Enzyme aus Tieren, Pflanzen, Mikroorganismen oder aus tierischen Organen, wie z. B. Schweineherz, eingesetzt werden, die in der Lage sind, alpha-Ketosäuren durch Transaminierung in natürliche L-Aminosäuren umzuwandeln.

Bevorzugt wird jedoch mit Mikroorganismen gearbeitet, die eine Transaminase besitzen, wie zum Beispiel Mikroorganismen der Gattungen Paracoccus, Alkaligenes, Rhizobium, Pseudomonas, Serratia, Agrobacterium, Streptomyces oder Enterobakterium.

Innerhalb dieser bevorzugten Gattungen können beispielsweise verwendet werden: Alcaligenes faecalis DSM 4115, Alcaligenes denitrificans DSM 4114, Pseudomonas paucimobilis DSM 4120, Pseudomonas spec. DSM 4119, Serratia plymuthica DSM 4116, Agrobacterium tumefaciens, Escherichia coli DH1, Enterobacter agglomerans DSM 4122, Enterobacter spec. DSM 4121 Streptomyces hygroscopicus, Streptomyces viridochromogenes oder die Bodenisolate DSM 4113, DSM 4117 und DSM 4118.

Besonders bevorzugt werden Escherichia coli ATCC 11303 und Paracoccus denitrificans DSM 65.

Die Mikroorganismen können als Rein- oder Mischkultur eingesetzt werden.

Diese Mikroorganismen sind bei der Deutschen Sammlung für Mikroorganismen und Zellkulturen (DSM), Mascheroder Weg 1B, 3300 Braunschweig, Deutschland, oder der American Type Culture Collection (ATCC); 12301 Parklawn Drive; Rockville, Maryland 20852; USA verfügbar.

Man kann die Enzymaktivitäten der Mikroorganismen steigern, indem man Stämme selektiert, die gegen das Produkt der erfindungsgemäßen Biotransformation 3-(2-Thienyl)-alanin (2A) resistent sind oder diese Verbindung als alleinige Stickstoffquelle nutzen.

Dies ist ein allgemein gebräuchliches Verfahren zur Selektion guter Produzenten für die Aminosäure L-Phenylalanin und wird beispielsweise in der japanischen Patentanmeldung JP 9071-698 beschrieben.

Ebenso können durch Selektion und Mutation, in an sich bekannter Weise, gegen steigende Mengen der 2-Hydroxy-3-thienylacrylsäuren oder deren Salzen in den Anzuchtmedien für die weiteren Arbeiten Mikroorganismen ausgewählt werden, die aufgrund ihrer Adaption an das Substrat die Biotransformation in höheren Ausbeuten durchführen.

Besonders hohe Ausbeuten werden erreicht, wenn gentechnisch erzeugte Mutanten von Mikroorganismen eingesetzt werden. Besonders bevorzugt wird mit dem Bakterienstamm E.coli ATCC 11303 gearbeitet, der mit einem das tyrB-Gen bzw. zusätzlich mit einem das aspA-Gen enthaltenden Plasmid transformiert wurde, wobei das tyrB-Gen für die aromatische Transaminase und das aspA-Gen für die Aspartase in E.coli kodiert. Die derart transformierten E.coli ATCC 11303 können beispielsweise nach den Deutschen Patentanmeldungen P 3631829.9 und P 3713755.2 sowie nach der EP 248 357 hergestellt werden.

Die Mikroorganismen werden vorteilhaft in einem für ihr Wachstum optimalen Nährmedium unter günstigen Temperatur- und Belüftungsbedingungen bis zu einem Trockengewicht von etwa 4 - 60 g/l Nährlösung angezogen. Die für den jeweiligen Mikroorganismus günstigsten Bedingungen sind dem Fachmann entweder bekannt oder können in einfachen Vorversuchen festgestellt werden. Die Zellen werden dann in der Nährlösung oder von der Nährlösung abgetrennt zur Biotransformation der 2-Hydroxy-3-thienylacrylsäuren eingesetzt. Die Biotransformation kann mit ganzen oder auch mit aufgeschlossenen Zellen durchgeführt werden, wobei die üblichen Aufarbeitungsmethoden eingesetzt werden.

Die Biotransformation kann auch mit Zellextrakten, isolierten Gesamtproteinen sowie gereinigten Transaminasen durchgeführt werden. Aus Gründen der Arbeitserleichterung wird bevorzugt mit intakten Zellen gearbeitet. Jedoch kann die Isolierung der Transaminasen aufgrund einer längeren Lebensdauer des Enzyms sowie einer besseren verfahrenstechnischen Steuerung der Reaktion ebenfalls vorteilhaft sein. Beispiele hierfür finden sich beispielsweise bei S.C.Ng und J. Baldwin, Bull. Sing. N. I. Chem. 18, 127 (1990), wobei eine Aspartat- Transaminase (AST) aus E.coli (I. G. Fotheringham, S. A. Dacey, P. P. Taylor, T. G. Smith, M. G. Hunter, M. G. Finlay, S. B. Primrose, D. M. Parker und R. M-Edwards, Biochem. J., 234, 593 (1986)) zur Synthese einer Reihe von L-alpha-Aminosäuren aus alpha-Ketosäuren beschrieben wird. Es ist weiterhin möglich, die Mikroorganismen oder die Enzyme in fixierter Form einzusetzen. Für die Fixierung kommen die bekannten Verfahren in Betracht, vorteilhaft die Methoden nach den Deutschen Offenlegungsschriften 3237341 und 3243591.

Als Träger der fixierten Enzyme wird bevorzugt Eupergit C®, VA-Epoxy, Kieselgele z. B. Grace XWP 250 UMP, XWP 300 MP, XWP 350 MP, XWP 1000 MP, XWP 1500 UMP, XWP 350 LP, XWP 350 HP, besonders bevorzugt XWP 500 MP eingesetzt.

Das immobilisierte Enzym oder das nicht-immobilisierte Enzym kann jeweils für das kontinuierliche Verfahren oder das Batchverfahren eingesetzt werden. Bevorzugt wird im Batchverfahren das immobilisierte Enzym verwendet.

Die Mikroorganismen bzw. das isolierte oder immobilisierte Enzym werden in der bevorzugten Ausführungsform in einem physiologischen Puffer suspendiert, unter Zugabe einer 2-Hydroxy -3-thienylacrylsäure (3) und des Aminogruppendonators. Je nach Menge der Mikroorganismen kann die dem Ansatz zugegebene enzymatische Aktivität in weiten Bereichen eingestellt werden. Zweckmäßig liegt sie zwischen 10 und 30.000 µmol/min l. Vorzugsweise enthält der Ansatz Zellmengen mit einer Enzymaktivität von 10.000 bis 20.000 µmol/min l.

Als Aminogruppendonator können Asparagin, Glutamin, Asparaginsäure und/oder Glutaminsäure, jeweils in der L-Form, oder Fumarat bzw. Fumarsäure in Kombination mit Ammoniumionen oder Harnstoff verwendet werden, vorzugsweise wird jedoch mit L-Asparaginsäure und L-Glutaminsäure, besonders bevorzugt mit L-Asparaginsäure gearbeitet. Diese Vorstufen werden in Form ihrer freien Säuren oder geeigneten Salzen ( entsprechend dem Medium) in mindestens äquimolaren Mengen bzw. im Überschuß zum Substrat (3) eingesetzt. Verhältnisse von 1:1 bis 5:1, vorteilhaft 1:1 bis 2:1, haben sich bewährt.

Beim Einsatz von Salzen werden natürliche Ionen gewählt, die die Enzymaktivität nicht nennenswert beeinflussen. Bevorzugt sind dies Natrium-, Kalium- und Ammoniumsalze.

Die Zugabe der Reaktionskomponenten zum Reaktionsansatz kann als Lösung in Wasser, mit Wasser mischbaren Lösemitteln, vorzugsweise reinem Methanol oder mit Wasser verdünntem Methanol jeder Verdünnungsstufe oder durch Zugabe der festen Substanzen gleichzeitig erfolgen. Bevorzugt ist jedoch eine chargenweise bzw. kontinuierliche Zugabe in Mengen von 0,5-10%, insbesondere 2-5%, jeweils bezogen auf das Gewicht des Reaktionsansatzes, über einen Zeitraum von 0,5-24 Stunden, vorzugsweise 8-16 Stunden. Es wird vorteilhaft bei einem pH-Wert zwischen 5 und 9, insbesondere zwischen 7 und 8.5 gearbeitet. Es ist außerdem zweckmäßig, die Biotransformation in einem Temperaturbereich von 10° - 65°C, insbesondere bei 30°- 45°C durchzuführen. Bei niedrigeren Temperaturen verläuft die Biotransformation zunehmend langsamer, während höhere Temperaturen zu fortschreitender Desaktivierung des Enzyms führen.

Es ist nicht notwendig, die Mikroorganismen vor bzw. während der Biotransformation zu permeabilisieren. Hohe Umsatzraten und Ausbeuten werden insbesondere dann erreicht, wenn der Reaktionsansatz während der Inkubation mit dem Mikroorganismus oder mit dem Enzym unter Ausschluß von Luftsauerstoff gehalten wird. Geeignet sind Inertgase wie Stickstoff sowie Edelgase, wie beispielweise Argon.

Bei der Durchführung der Transaminierung mittels immobilisierter Enzyme wird die Reaktionslösung chargenweise oder kontinuierlich mit dem Katalysator umgesetzt. Die Konzentration an 2-Hydroxy-3-(2-thienyl)-acrylsäure liegt in der Reaktionslösung bei 0,5 - 5 %, vorzugsweise bei 1 - 2 %. Die Konzentration der Donoraminosäure entspricht dem Ansatz mit ganzen Zahlen. Die Reaktionslösung (gekühlt vorgelegt 4° C) wird bei 10 - 65° C, vorzugsweise bei 30 - 45° C umgesetzt. Das immobilisierte Enzym läßt sich sowohl im Rührreaktor als auch im Säulenreaktor, vorzugsweise im Festbettverfahren, einsetzen. Bei letzterem Verfahren wird die Reaktionslösung mit einer Geschwindigkeit von 0,1 - 20 ml, insbesondere 0,1 - 0,5 ml pro Min., über die Säule gepumpt.

Die nicht-proteinogenen L-Thienylalanine können als Baustein für die Synthese von Peptiden, vorzugsweise für die Synthese von Bradykinin oder Bradykinin verwandten Peptiden verwendet werden.

Bradykinin hat die Aminosäuresequenz Arg-Pro-Pro-Gly-Phe-Ser-Pro-Phe-Arg und gehört zu den Kininen, einer Gruppe von Plasmahormonen. Ein Bradykininverwandtes Peptid ist das Lysylbradykinin, das - ebenso wie das Bradykininblutdrucksenkend wirkt, die Kontraktion spezifischer Muskeln stimuliert und schmerzstimulierend wirkt (Concise Encyclopedia Biochemistry, 2. Ausgabe, Thomas Scott and Mary Eagleson, Walter de Gruyter, Berlin, New York 1988, S. 75).
Peptide der Formel A-B-C-E-F-K-(D)-Tic-G-M-F'-I, worin A für Wasserstoff, Alkyl, Alkanoyl, Alkoxycarbonyl, Alkylsulfonyl, Cycloalkyl, Aryl, Arylsulfonyl, Heteroaryl oder eine Aminosäure steht, die gegebenenfalls substituiert sein können, B eine basische Aminosäure ist, C ein Di- oder Tripeptid bedeutet, E für den Rest einer aromatischen Aminosäure steht, F unabhängig voneinander eine gegebenenfalls in der Seitenkette substituierte Aminosäure ist, F' wie F definiert ist, -NH-(CH₂)₂₋₈ oder gegebenenfalls eine direkte Bindung bedeuten kann, -OH, -NH₂ oder -NHC₂H₅ ist und Keinen Rest -NH-(CH₂)₁₋₄-CO- bedeutet oder für eine direkte Bindung steht, haben bradykininantagonistische Wirkung. Ihr therapeutischer Nutzen umfaßt alle pathologischen Zustände, die durch Bradykinin und Bradykinin-verwandte Peptide vermittelt, ausgelöst oder unterstützt werden.

Die Peptide werden nach allgemein bekannten Methoden der Peptidchemie, siehe z. B. Houben-Weyl, Methoden der organischen Chemie, Band 15/2, bevorzugt mittels Festphasensynthese wie z. B. von B. Merrifield, J. Am. Chem. Soc. 85, 2149 (1963) oder R.C. Sheppard, Int. J. Peptide Protein Res. 21, 118 (1983) beschrieben oder durch äquivalente bekannte Methoden hergestellt. Als α-Aminoschutzgruppe werden Urethanschutzgruppen wie z. B. die tert.-Butyloxycarbonyl(Boc)- oder Fluorenylmethyloxycarbonyl(Fmoc)-Schutzgruppe verwendet. Falls zur Verhinderung von Nebenreaktionen oder für die Synthese spezieller Peptide erforderlich, sind die funktionellen Gruppen in der Seitenkette von Aminosäuren durch geeignete Schutzgruppen (siehe z. B. T.W. Greene, "Protective Groups in Organic Synthesis") zusätzlich geschützt.

Die anschließenden Beispiele dienen dazu, die Erfindung weiter zu erläutern.

### Beispiel 1: Synthese von 2-Hydroxy-3-(2-thienyl)-acrylsäure (3A)

### Synthese nach der Azlacton-Route

### Azlactonsynthese und Hydrolyse zu 2-Hydroxy-3-(2-thienyl)-acrylsäure (3A)

420 g (4,9 mol) wasserfreies Natriumacetat und 500 g Acetylglycin werden in 1300 g Acetanhydrid gelöst bzw. suspendiert und mit 719 g Thiophen-2-Aldehyd versetzt. Unter Inertgas wird der Ansatz zum Sieden erhitzt und 1,5 Std. unter Rückfluß gekocht. Anschließend wird im Eisbad gekühlt und das ausgefallene Produkt abgesaugt. Man wäscht viermal mit 300 ml Eiswasser und trocknet den Rückstand im Vakuumtrockenschrank bei 50°C. Ausgefallenes Produkt aus den vereinigten Filtraten wird in gleicher Weise behandelt. Gesamtausbeute an Azlacton (7 A): 730 g, 88%.
Schmelzpunkt: 137-140°C
¹H-NMR (100 MHz, chem. Verschiebung in ppm, DMSO-d₆):
   8,2 - 6,8 (mehrere m, 4 H, aromat. H, =CH);
   4,60 (s, 3H, CH₃)

193 g (1 mol) des so erhaltenen Azlactons (7 A) werden in 1,5 l siedende Salzsäure eingetragen. Nach 30 Min. Rückfluß ist ein Großteil des Eduktes gelöst. Man filtriert heiß ab und wäscht mit wenig Wasser nach. Der rotbraune Rückstand besteht aus der Aminoacetylsäure 8 A (55 g, 26%). Das Filtrat wird unter Rühren auf 0°C abgekühlt. 2-Hydroxy-3-(2-thienyl)-acrylsäure (3A) und weitere Aminoacetylsäure 2-Acetoamido-acrylsäure 8A fallen aus. Man saugt erneut ab und wäscht mit wenig kaltem Wasser nach. Der auf der Fritte verbleibende Rückstand wird mehrfach mit Ether digeriert und abgesaugt. Auf der Fritte verbleibt die Aminoacetylacrylsäure (8A; 50 g, 25%).

Aus der Etherphase wird nach Einrotieren und Trocknen die 2-Hydroxy-3-(2-thienyl)-acrylsäure (3 A) isoliert (46g, 27%).
Schmelzpunkt: 166-168°C
¹H-NMR (100 MHz, chem. Verschiebung in ppm, DMSO-d₆):
   7,52; 7,25; 7,02 (3 dd, 3 H, aromat. H)
   6,75 (s, 1 H, =CH)

### Alternativroute: Umsetzung des Azlactons (7 A) nach Isolierung der 2-Aminoacetylacrylsäure (8 A) zur 2-Hydroxy-3-(2-thienyl)-acrylsäure (3 A)

96,5 g des Azlactons (7 A) werden in 500 ml Dioxan und 11 ml Wasser gelöst: Man leitet anschließend HCl-Gas ein. Aminoacetylacrylsäure beginnt sich kristallin abzuscheiden und liegt bei Beendigung der Reaktion nach 40 Min. als dicke Suspension vor. Unter Rühren gibt man 11 Diethylether hinzu und vertreibt durch Einleitung von Stickstoff den gelösten Chlorwasserstoff. Man saugt ab, wäscht gut mit Ether nach und befreit im Vakuum von Lösemittelresten. Ausbeute an Aminoacetylacrylsäure: 99,5 g, 94% d.Th.
Schmelzpunkt: 232-235°C
¹H-NMR (100 MHz, chem. Verschiebung in ppm, DMSO-d₆):
   9,25 (s, 1 H, NH)
   7,75 (d, 1 H, aromat. H)
   7,70 (s, 1 H, =CH)
   7,50 (d, 1 H, aromat. H)
   7,10 (dd, 1 H)

Die sich anschließende Hydrolyse mit Salzsäure wird wie oben beschrieben durchgeführt; Ausbeute an 2-Hydroxy-3-(2-thienyl)-acrylsäure 70-75%.

### Synthese nach der Hydantoin-Route

### Umsetzung von 5-(2-Thiophenal)-hydantoin (6 A) mit Natronlauge zu 2-Hydroxy-3-(2-thienyl)-acrylsäure (3A)

Unter Rühren und Inertgaseinleitung werden 1,2 l 5N NaOH zum Sieden erhitzt. Man trägt 77,7 g (0,4 mol) des Hydantoins (6 A) ein und beläßt für 60 Min. unter Rückfluß. Anschließend wird im Eisbad gekühlt und langsam mit 500 ml konz. Salzsäure versetzt. Ein Teil des Produktes (3 A) fällt direkt aus, der Rest kann mit Ether aus dem wässrigen Filtrat extrahiert werden. Gesamtausbeute an getrocknetem Produkt 3 A: 66,9% d. Th.

### Analytische Daten des Hydantoins (6A):

Schmelzpunkt: 263-264°C
¹H-NMR (100 MHz, chem. Verschiebung in ppm, DMSO-d₆):
   7,70; 7,60; 7,18 (3 dd, 3 H, aromat. H)
   6,59 (s, 1 H, =C-H)

### Beispiel 2: Synthese von 2-Hydroxy-3-(3-thienyl)-acrylsäure (3B)

Die Synthese erfolgt wie in Beispiel 1 beschrieben.

### Analytische Daten:

a) 5-(3-Thiophenal)-2-methyl-3-oxazolin-4-on (7B):
   Schmelzpunkt 112-116° C
   ¹H-NMR (100 MHz, chem. Verschiebung in ppm, DMSO-d₆):
      8,34; 7,90; 7,68 (3 dd, 3 H, aromat. H )
      7,28 (s, 1 H, =CH)
      2,37 (s, 1 H, -CH₃)
b) 2-Aminoacetyl-3-(3-thienyl)-acrylsäure (8 B):
   Schmelzpunkt 205 ° C
   ¹H-NMR (100 MHz, chem. Verschiebung in ppm, DMSO-d₆):
      7,93; 7,60; 7,41 (3 dd, 3 H, aromat. H)
      7,33 (s, 1 H, =CH)
      2,00 (s, 1 H, CH₃N-Ac)
c) 5-(3-Thiophenal)-hydantoin (6 B):
   Schmelzpunkt 270 ° C
   ¹H-NMR (100 MHz, chem. Verschiebung in ppm, DMSO-d₆):
      7,95 (m, 1 H, aromat. H)
      7,68 - 7,42 (4dd, 2 H, aromat. H)
      6,50 (s, 1 H, =CH)
d) Hydroxy-3-(3-thienyl)-acrylsäure (3 B):
   Schmelzpunkt 178 - 180° C
   ¹H-NMR (100 MHz, chem. Verschiebung in ppm, DMSO-d₆):
      9,10 (s, 1 H, COOH)
      7,75; 7,45 (2 m, 3 H, aromat. H)
      6,50 (s, 1 H, =CH)

### Beispiel 3: Synthese von 2-Hydroxy-3-(4-brom-3-thienyl)-acrylsäure (3C)

Die Synthese erfolgt wie in Beispiel 1 beschrieben.

### Analytische Daten:

a) 5-(4-Brom-2-thiophenal)-2-methyl-3-oxazolin-4-on (7C):
   Schmelzpunkt 142-145° C
   ¹H-NMR (100 MHz, chem. Verschiebung in ppm, DMSO-d₆):
      8,05; 7,75; 7,54 (je s, je 1 H, aromat. H, =CH)
      2,37 (s, 3 H, CH₃)
b) 2-Aminoacetyl-3-(4-brom-2-thienyl)-acrylsäure (8C):
   Schmelzpunkt 213-215° C
   ¹H-NMR (100 MHz, chem. Verschiebung in ppm, DMSO-d₆):
      7,30 (s, br, 1 H, NH)
      7,80; 7,65; 7,5 (je s, je 1 H, aromat. H, =CH)
      2,00 (s, 3 H, CH₃N-Ac)
c) 5-(4-Brom-2-thiophenal)-hydantoin (6C):
   Schmelzpunkt 220-221° C
   ¹H-NMR (100 MHz, chem. Verschiebung in ppm, DMSO-d₆):
      11,35; 10,52 (2 s, 2 H, NH)
      7,77; 7,65 (2 "s", 2 H, aromat. H)
      6,48 (s, 1 H, =CH)
d) 2-Hydroxy-3-(4-brom-2-thienyl)-acrylsäure (3C):
   Schmelzpunkt 194-195° C
   ¹H-NMR (100 MHz, chem. Verschiebung in ppm, DMSO-d₆):
      9,30 (s, br, 1 H, COOH)
      7,63; 7,25 (je s [br], je 1 H, aromat. H)
      6,75 (s, 1 H, =CH)

### Beispiel 4: Synthese von 2-Hydroxy-3-(5-brom-2-thienyl)-acrylsäure (3D)

Die Synthese erfolgt wie in Beispiel 1 beschrieben.

### Analytische Daten:

a) 5-(5-Brom-2-thiophenal)-2-methyl-3-oxazolin-4-on (7D):
   Schmelzpunkt 192° C
   ¹H-NMR (100 MHz, chem. Verschiebung in ppm, DMSO-d₆):
      7,55 (d, 1 H, aromat. H)
      7,50 (s, 1 H, CH)
      7,35 (d, 1 H, aromat. H)
      2,35 (s, 3 H, CH₃)
b) 2-Aminoacetyl-3-(5-brom-2-thienyl)-acrylsäure (8D):
   Schmelzpunkt 221-222° C
   ¹H-NMR (100 MHz, chem. Verschiebung in ppm, DMSO-d₆):
      9,30 (s, 1 H, NH)
      7,75 (s [br], 1 H, =CH)
      7,35 (d, 1 H, aromat. H)
      7,25 (d, 1 H, aromat. H)
      2,03 (s, 3 H, N-Ac)
c) 5-(5-Brom-2-thiophenal)-hydantoin (6 D):
   Schmelzpunkt 235-236° C
   ¹H-NMR (100 MHz, chem. Verschiebung in ppm, DMSO-d₆):
      11,3; 10,4 (2 s, 2 H, NH)
      7,42; 7,30 (2 d, 2 H, aromat. H)
      6,52 (s, 1 H, =CH)
d) 2-Hydroxy-3-(5-brom-2-thienyl)-acrylsäure (3 D):
   Schmelzpunkt 56-58° C
   ¹H-NMR (100 MHz, chem. Verschiebung in ppm, DMSO-d₆):
      9,40 (s [br], 1 H, COOH)
      7,05 (d, 1 H, aromat. H)
      6,75 (d [aufgesp.], 1 H, aromat. H)
      6,70 (s, 1 H, =CH)

### Beispiel 5: Synthese von 2-Hydroxy-3-(3-methyl-2-thienyl)-acrylsäure (3E)

Die Synthese erfolgt wie in Beispiel 1 beschrieben.
a) 5-(3-Methyl-2-thiophenal)2-methyl-3-oxazolin-4-on (7 E):
   Schmelzpunkt 145-146° C
   ¹H-NMR (100 MHz, chem. Verschiebung in ppm, DMSO-d₆):
      7,90 (d, 1 H, aromat. H)
      7,35 (s, 1 H, =CH)
      7,03 (d, 1 H, aromat. H)
      2,40; 2,25 (2 s, 6 H, CH₃)
b) 2-Aminoacetyl-3-(3-methyl-2-thienyl)-acrylsäure (8 E):
   Schmelzpunkt 231-232° C
   ¹H-NMR (100 MHz, chem. Verschiebung in ppm, DMSO-d₆):
      9,25 (s, br, 1 H, NH)
      7,65 (d, 1 H, aromat. H)
      7,60 (s, 1 H, =CH)
      7,00 (d, 1 H, aromat. H)
c) 5-(3-Methyl-2-thiophenal)-hydantoin (6 E):
   Schmelzpunkt-212-214° C
   ¹H-NMR (100 MHz, chem. Verschiebung in ppm, DMSO-d₆):
      11,23; 10,02 (2 s, 2 H, NH)
      7,65; 7,00 (2 d, 2 H, aromat. H)
      6,60 (s, 1 H, =CH)
      2,29 (s, 3 H, CH₃)
d) 2-Hydroxy-3-(3-methyl-2-thienyl)-acrylsäure (3 E):
   Schmelzpunkt 184-185° C
   ¹H-NMR (100 MHz, chem. Verschiebung in ppm, DMSO-d₆):
      7,45 (d, 1 H, aromat. H)
      6,90 (d, 1 H, aromat. H)
      6,70 (s, 1 H, =CH)
      2,23 (s, 3 H, CH₃)

### Beispiel 6: Synthese von 2-Hydroxy-3-(5-methyl-2-thienyl)-acrylsäure (3 F)

Die Synthese erfolgt wie in Beispiel 1 beschrieben.

### Analytische Daten:

a) 5-(5-Methyl-2-thiophenal)-2-methyl-3-oxazolin-4-on (7 F):
   Schmelzpunkt 280° C (Zers.)
   ¹H-NMR (100 MHz, chem. Verschiebung in ppm, DMSO-d₆):
      7,57 (d, 1 H, aromat. H)
      7,48 (s, 1 H, =CH)
      6,93 (d, aufgesp., 1 H, aromat. H)
      2,50; 2,32 (2 s, je 3 H, CH₃)
b) 2-Aminoacetyl-3-(5-methyl-2-thienyl)-acrylsäure (8 F):
   Schmelzpunkt 257° C
   ¹H-NMR (100 MHz, chem. Verschiebung in ppm, DMSO-d₆):
      9,20 (s, br, 1 H, NH)
      7,60 (s, 1 H, =CH)
      7,30 (d, 1 H, aromat. H)
      6,80 (d. aufgesp., 1 H, aromat. H)
      2,00 (s, 3 H, N-Ac)
c) 5-(5-Methyl-2-thiophenal)-hydantoin (6 F):
   Schmelzpunkt 263-264° C
   ¹H-NMR (100 MHz, chem. Verschiebung in ppm, DMSO-d₆):
      11,2; 10,22 (2 s, 2 H, NH)
      7,40; 6,85 (2 d, 2 H, aromat. H)
      6,45 (s, 1 H, =CH)
      2,45 (s, 3 H, -CH)
d) 2-Hydroxy-3-(5-methyl-2-thienyl)-acrylsäure (3 F):
   Schmelzpunkt 184-185° C
   ¹H-NMR (100 MHz, chem. Verschiebung in ppm, DMSO-d₆):
      9,3 (s, br, 1 H, COOH)
      7,05 (d, 1 H, aromat. H)
      6,7 (d. aufgesp., 1 H, aromat. H)
      6,68 (s, 1 H, =CH)
      2,43 (s, 3 H, CH₃)

### Beispiel 7: Synthese von 2-Hydroxy-3-(5-nitro-2-thienyl)-acrylsäure (3G)

Die Synthese erfolgt wie in Beispiel 1 beschrieben.
a) 5-(5-Nitro-2-thiophenal)-2-methyl-3-oxazolin-4-on (7 G):
   Schmelzpunkt > 300° C
   ¹H-NMR (100 MHz, chem. Verschiebung in ppm, DMSO-d₆):
      8,05 (d, 1 H, aromat. H)
      7,68 (s, 1 H, =CH)
      7,48 (d, 1 H, aromat. H)
      2,16; 2,05 (2 S, 3 H, CH₃)
b) 2-Aminoacetyl-3-(5-nitro-2-thienyl)-acrylsäure (8 G):
   Schmelzpunkt 220° C
   ¹H-NMR (100 MHz, chem. Verschiebung in ppm, DMSO-d₆):
      8,15 (d, 1 H, aromat. H)
      7,75 (s, 1 H, =CH)
      7,60 (d, 1 H, aromat. H)
      2,06 (s, 1 H, CH₃, N-Ac)
c) 5-(5-Nitro-2-thiophenal)-hydantoin (6 G):
   Schmelzpunkt: nicht bestimmt
   ¹H-NMR (100 MHz, chem. Verschiebung in ppm, DMSO-d₆): nicht bestimmt
d) 2-Hydroxy-3-(5-nitro-2-thienyl)-acrylsäure (3 G):
   Schmelzpunkt 192-193° C
   ¹H-NMR (100 MHz, chem. Verschiebung in ppm, DMSO-d₆):
      8,06 (d, 1 H, aromat. H)
      7,34 (d, 1 H, aromat. H)
      6,87 (s, 1 H, =CH)

### Beispiel 8: Synthese von L-3-(2-Thienyl)-alanin (2A)

30 g 2-Hydroxy-3-(2-thienyl)-acrylsäure, 29 g L-Aspartat und 40 mg Pyridoxalphosphat wurden in 700 ml Aqua dest. gelöst und mit NaOH ein pH-Wert von 8 eingestellt (Lösung 1). Die erhaltene Lösung wurde auf 4° C gekühlt. In einem 11 Reaktor wurden 100 ml einer Suspension von E. coli Z 1196/6 (entspricht ca. 6 g Trockenmasse oder 3000 u Transaminaseaktivität) mit 200 ml Aqua dest. verdünnt vorgelegt (Lösung 2). Der pH-Wert dieser Suspension wurde ebenfalls auf pH 8 eingestellt und das Gemisch auf 40° C temperiert. Begast wurde mit 0,1 vvm N₂. Zu dieser Suspension wurde unter Rühren Lösung 1 im Verlauf von 16 Std. zudosiert. Der pH-Wert wurde mittels NaOH/H₂SO₄ konstant gehalten.
Die Konzentration an L-3-(2-Thienyl)-alanin betrug nach 24 Std. 24,6 g/l (82 % Ausbeute).
Die Reaktionsmischung wurde zentrifugiert (15 Min., 8000 g), der Überstand auf pH 1,5 eingestellt, mit 5 g Aktivkohle versetzt und die Suspension 30 Min. bei 70° C gerührt. Anschließend wurde auf 4° C gekühlt und der Niederschlag abfiltriert. Das Filtrat wurde mit NaOH auf pH 12 eingestellt.
Die Lösung wurde auf eine Säule - gefüllt mit 11 Dowex 1 x 2 (Cl) - aufgetragen. Gespült wurde mit 2 l Wasser (mit Ammoniak auf pH 9 eingestellt). L-3-(2-Thienyl)-alanin wurde mit 4 l 0,l05 % Essigsäure eluiert. Das Eluat wurde im Vakuum auf 300 ml eingeengt und das entstandene Konzentrat lyophilisiert.
- Ausbeute L-3-(2-Thienyl)-alanin:: 17,8 g.
- HPLC:: Reinheit > 98 %, D-Enantiomer < 0,2 %
- Drehwert:: -30,1 ° (c= 1, H₂O)
- ¹H-NMR: (300 MHz, in D₂O, chem. Verschiebung in ppm):
7,26 (d, 1 H, aromat. H)
6,88 (m, 2 H, aromat. H)
3,87 ("t", 1 H, =CH)
3,35 (m, 2 H, CH₂)
- ¹³C-NMR: (75 MHz, in D₂O, chem. Verschiebung in ppm):
174 (COOH)
136, 128, 128, 126 (aromat.)
56 (=CH)
30 (CH₂)

### Beispiel 9: Synthese von L-3-(3-Thienyl)-alanin (2B)

Die Durchführung entsprach weitgehend Beispiel 8. Lösung 1 enthielt jedoch nur 20 g Hydroxy-3-(3-thienyl)-acrylsäure und 19 g L-Aspartat. Die Konzentration an Produkt betrug 11,5 g/l (58 %) nach der Umsetzung. Nach der Ionenaustauscherchromatographie wurden 7,9 g (40 %) L-3-(-3-Thienyl)-alanin (2B) erhalten.
- HPLC:: Reinheit > 90 %, D-Enantiomer < 0,2 %
- Drehwert:: -38,9° (C = 1, H₂O)
- ¹H-NMR: (300 MHz, in D₂O, chem. Verschiebung in ppm):
7,38; 7,18; 6,97 (3 s, br, 3 H, aromat. H)
3,88 ("t", 1 H, =CH)
3,16 (m, 2 H, CH₂)

### Beispiel 10: Synthese von L-3-(4-Brom-2-thienyl)-alanin (2C)

Die Umsetzung wurde analog Beispiel 8 durchgeführt. Jedoch wurde im kleineren Maßstab gearbeitet und die Isolierung modifiziert.
Lösung 1: 750 mg (3C), 720 mg L-Aspartat, 4 mg Pyridoxalphosphat ad 35 ml Aqua dest. Lösung 2 : 5 ml Zellsuspension + 10 ml Aqua dest.
Nach 6 Std. Reaktionszeit waren 60 mg L-3-(4-Brom-2-thienyl)-alanin gebildet worden. Nach Abtrennung der Zellen und Entfärbung mit Aktivkohle wurde die Lösung neutralisiert und das Volumen im Vakuum auf 10 ml reduziert.
Die Reinigung erfolgte mittels präparativer HPLC an einer Nucleosil C₁₈-Säule (10 cm x 40 x 250 mm); Fluß 20 ml/min⁻¹; Laufmittel A: H₂O, Laufmittel B: Acetonitril; Detektion 254 nm. Gradient 0 % B auf 20 % B in 80 Minuten, anschließend in 60 Minuten auf 50 % B. Die (2C) enthaltenden Fraktionen wurden vereinigt und im Vakuum auf 10 ml wäßrigen Rückstand eingeengt. Diese Lösung wurde lyophilisiert. Ausbeute 70 mg (10 %) (2C) erhalten.
- HPLC:: Reinheit > 95 %, D-Enantiomer < 0,2 %
- Drehwert:: -29,3° (c = 1, H₂O)
- ¹H-NMR:: (300 MHz, in D₂O, chem. Verschiebung in ppm):
7,40; 7,00 (2 s, 2 H, aromat. H)
4,00 ("t", 1 H, =CH)
3,42 (m, 2 H, CH₂)
- ¹³C-NMR:: (75 MHz, in D₂O, chem. Verschiebung in ppm):
173 (COOH)
138, 130, 124, 109 (aromat.)
56 (=CH)
30 (CH₂)

### Beispiel 11: Synthese von L-3-(5-Brom-2-thienyl)-alanin (2D)

Die Umsetzung wurde analog zu Beispiel 10 durchgeführt.
Jedoch setzt sich Lösung 1 wie folgt zusammen: Lösung 1: 1 g 2-Hydroxy-3-(brom-2-thienyl)-acrylsäure, 0,97 g L-Aspartat, 4 mg Pyridoxalphosphat ad 35 ml H₂O. Lösung 2: 5 ml Zellsuspension + 10 ml Aqua dest. Nach 6 Std. Reaktionszeit wurden 43 mg (4,3 %) Produkt detektiert. Nach präparativer HPLC (Beispiel 10) wurden 27 mg (2,7 %) L-3-(5-Brom-2-thienyl)-alanin erhalten.
- HPLC:: Reinheit > 85 % D-Enantiomer < 0,2 %
- ¹H-NMR:: (300 MHz, in D₂O, chem. Verschiebung in ppm):
7,50 - 6,95 (2 m, 2 H, aromat. H)
3,85 (m, 1 H, =CH)
3,38 (m, 2 H, CH₂)
- ¹³C-NMR:: (75 MHz, in D₂O, chem. Verschiebung in ppm):
133; 132; 130; 128 (aromat.)
60 (=CH)
23 (CH₂)

### Beispiel 12: Synthese von L-3-(3-Methyl-2-thienyl)-alanin (2E)

Die Umsetzung wurde analog zu Beispiel 10 durchgeführt. Lösung 1 setzt sich jedoch wie folgt zusammen: Lösung 1: 300 mg 2-Hydroxy-3-(5-methyl-2-thienyl)-acrylsäure, 290 mg L-Aspartat, 4 mg Pyridoxalphosphat ad 35 ml H₂O. Lösung 2: 5 ml Zellsuspension + 10 ml Aqua dest. Nach 6 Std. Reaktionszeit wurden 210 mg (70 %) Produkt detektiert. Nach präparativer HPLC (Beispiel 10) wurden 184 mg (61 %) L-3-(3-Methyl-2-thienyl)-alanin erhalten.
- HPLC:: Reinheit > 95 %, D-Enantiomer < 0,2 %
- Drehwert:: -8,8° (C = 1,1, 1 N NaOH)
- ¹H-NMR:: (300 MHz, in D₂O, chem. Verschiebung in ppm):
7,18; 6,82 (2 d, 2 H, aromat. H)
3,78 (dd, 1 H, =CH)
3,23 (2 dd, 2 H, CH₂)
2,10 (s, 3 H, CH₃)
- ¹³C-NMR:: (75 MHz, in D₂O, chem. Verschiebung in ppm):
175 (COOH)
137; 131; 130; 124 (aromat.)
56 (=CH)
29 (CH₂)
13 (CH₃)

### Beispiel 13: Synthese von L-3-(5-Methyl-2-thienyl)-alanin (2F)

Die Umsetzung wurde analog zu Beispiel 10 durchgeführt. Lösung 1: 300 mg 2-Hydroxy-3-(5-methyl-2-thienyl)-acrylsäure, 290 mg L-Aspartat, 4 mg Pyridoxalphosphat ad 35 ml H₂O.
Lösung 2: 5 ml Zellsuspension + 10 ml Aqua dest. Nach 6 Std. Reaktionszeit wurden 210 mg (70 %) Produkt detektiert. Nach präparativer HPLC (Beispiel 10) wurden 150 mg (50 %) L-3-(5-Methyl-2-thienyl)-alanin erhalten.
- HPLC:: Reinheit > 95 %, D-Enantiomer < 0,2 %
- Drehwert:: -19,6° (C = 1, H₂O)
- ¹H-NMR:: (300 MHz, in D₂O, chem. Verschiebung in ppm):
7,30; 6,92 (2 d, 2 H, aromat. H)
3,94 (m, 1 H, =CH)
3,40 (m, 2 H, CH₂)
2,20 (s, 3 H, Ch₃)
- ¹³C-NMR: (75 MHz, in D₂O, chem. Verschiebung in ppm):
212 (COOH)
140; 135; 134; 128 (aromat.)
59 (=CH)
32 (CH₂)
17 (CH₃)

### Beispiel 14: Bestimmung der Enantiomerenreinheit mittels HPLC

- Laufmittel:: A) 12,5 mM Phosphatpuffer pH 7,2
B) ACN
- Reagenzien:: 1) 50 mg/ml OPA in EtOH p.A.
2) 100 mg/ml Boc-L-Cystein in EtOH p.A.
3) 1 M Kaliumboratpuffer pH 10,4
Unmittelbar vor Gebrauch werden 980 µl 3) mit je 10 µl 1) und 2) versetzt. Die Lösung ist ca. 48 Std. bei 4° C stabil.
- Derivatisierung:: 10 µl einer geeigneten verdünnten Probe werden mit 90 µl Reagenz vermischt. Nach 120 Sek. kann die Probe injiziert werden.
- Säule:: Grom Amino-OPA (150 x 4,6 mm) mit identischer Vorsäule (20 x 4,6 mm);
- Fluß:: 1,5 mlmin⁻¹; Detektion 340 nm; Gradient: In 13 Minuten von 10 % auf 50 % B.

### Beispiel 15: Isolierung und Immobilisierung der Aromatische-Aminosäuren-Aminotransferase aus E. coli Z 1196/9

### Enzymisolierung:

20 g Zellen (E. coli Z 1196/9) wurden in 60 ml Puffer (20 mM KHPO₄/KH₂PO₄, 10 µM Pyridoxalphosphat, 5 mM 2-Mercaptoethanol, 10 mM EDTA und 12 mg Lysozym) suspendiert und 10 Minuten bei 30° C schwach gerührt. Die entstandene Suspension wurde 15 Minuten bei 4° C zentrifugiert und der klare Überstand für die weitere Aufarbeitung eingesetzt. Zunächst wurden mit 30 % Ammoniumsulfat 1 h bei 4° C gefällt und anschließend 15 Minuten bei
10.000 g / 4° C zentrifugiert. Der Niederschlag wurde verworfen und der Überstand einer Fällung mit 70 % Ammoniumsulfat (1 h / 4° C) unterworfen und die Suspension wie oben zentrifugiert. Der Niederschlag enthielt den größten Teil der Transaminase und wurde im Kupplungspuffer (s. u.) aufgenommen.

### Aktivierung des Kieselgels:

10 g Träger (Kieselgel XWP 50 MP von Grace) wurde in 100 ml Aqua dest. suspendiert und 2300 mg Aminopropyltriethoxysilan dazugegeben. Der pH-Wert wurde mit HCl auf 2,5 eingestellt und das Gemisch 2 h auf 65° C erwärmt. Anschließend wurde filtriert und mit Wasser bis zur Neutralität gewaschen und das Kieselgel 24 h bei 90° C getrocknet.
Der trockene Träger wurde in 160 ml 0,25 M KPP-Puffer pH 8 mit 2 % Glutardialdehyd aufgenommen und 2 h im Wasserstrahlvakuum unter leichtem Rühren inkubiert. Anschließend wurde wieder filtriert und gründlich mit Wasser gewaschen.

### Kupplung des Enzyms:

Der aktivierte Träger wurde in 200 ml 1M KPP-Puffer und 10 µM Pyridoxalphosphat (Kupplungspuffer) mit 130 mg / 10.000 u Enzym aufgenommen und über Nacht bei 4° C inkubiert, die Kupplungsausbeute lag bei etwa 80 %. Dieses Material wurde für Transaminierungen wie in Beispiel 16 beschrieben eingesetzt.

### Beispiel 16: Kontinuierliche Synthese von L-3-(2-Thienyl)-alanin mit immobilisiertem Enzym

10 g 2-Hydroxy-3-(2-thienyl)-acrylsäure` 9,3 g L-Asparaginsäure, 20 mg Pyridoxalphosphat und 1 ml Mercaptoethanol wurden in 1000 ml Aqua dest. gelöst und mit NaOH ein pH-Wert von 8 eingestellt. Die Lösung wird auf 4° C gekühlt und für die Reaktion eingesetzt.
Als Katalysator kam angereichertes Enzym (Aromatische-Aminosäuren-Aminotransferase aus E. coli Z 1196/9) immobilisiert an einen Kieselgelträger (Grace XWP 500 MP, 0,5 - 1 mm) mit einer spez. Aktivität von 500 - 800 u pro Gramm trockenem Träger zum Einsatz. 10 ml des Immobilisats wurden in einer Säule (40 x 18 mm Id.) vorgelegt, die mit einem Heizmantel auf 40° C temperiert wurde. Die Reaktionslösung wurde mit einem Fluß von 1,5 mlh⁻¹ durch die Säule gepumpt und wieder auf 4° C gekühlt. Die erzielte Raum-Zeit-Ausbeute lag bei 800 mgl⁻¹h⁻¹, bei einem Umsatz von 50 - 60 %. Diese Werte wurden kontinuierlich über 800 Stunden erzielt. Die Isolierung des L-3-(2-Thienyl)-alanin erfolgte analog zu Beispiel 8.

## Patentansprüche

1. Verfahren zur Herstellung von L-Thienylalaninen der Formel I 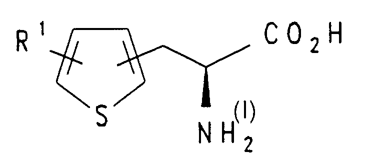 in welcher R¹ Wasserstoff, einen Halogen-, Nitro-, einen geradkettigen oder verzweigten Alkylrest bedeutet bzw. die Salze dieser Verbindungen, das dadurch gekennzeichnet ist, daß die Verbindungen der Formel II 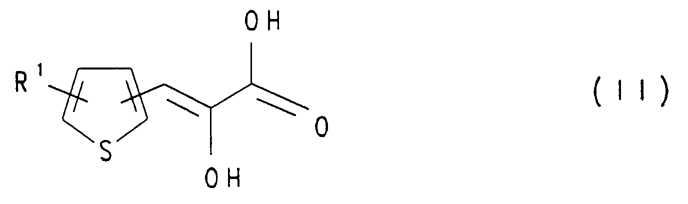 in welcher R¹ die obengenannten Bedeutungen hat und der 2-Hydroxyacrylsäurerest in der 2- oder 3-Stellung des Thiophenrings steht in Gegenwart der Aminosäuren Asparagin, Glutamin, L-Asparaginsäure und/oder L-Glutaminsäure und in Anwesenheit eines oder mehrerer Mikroorganismen und/oder Enzymen mit Transaminaseaktivität biotransformiert werden.

2. Verfahren nach Anspruch 1, dadurch gekennzeichnet, daß der Mikroorganismus und/oder das Enzym immobilisiert am Träger verwendet wird.

3. Verfahren nach Anspruch 1, dadurch gekennzeichnet, daß E.coli ATCC 11303 und/oder Paracoccus denitrificans DSM 65 eingesetzt werden.

4. Verfahren nach Anspruch 2, dadurch gekennzeichnet, daß die Biotransformation mit E.coli ATCC 11303, transformiert mit einem extrachromosomalen Element, welches das aus E.coli ATCC 11303 isolierte tyrB-Gen enthält, durchgeführt wird.

5. Verfahren nach Anspruch 1, dadurch gekennzeichnet, daß die Biotransformation mit Zellextrakt, isoliertem Gesamtprotein oder gereinigtem Enzym durchgeführt wird.

6. Verfahren nach Anspruch 1, dadurch gekennzeichnet, daß die aus Hydantoinderivaten oder aus Azlactonderivaten gewonnenen 2-Hydroxy-3-thienylacrylsäuren für die Biotransformation eingesetzt werden.

7. Verfahren nach Anspruch 1, dadurch gekennzeichnet, daß Aminogruppendonator und die betreffende 2-Hydroxy-3-thienylacrylsäure im Verhältnis 1:1 bis 5:1 eingesetzt werden.

8. Verfahren nach einem oder mehreren der Ansprüche 1 und 3-7, dadurch gekennzeichnet, daß die Biotransformation beim einem pH-Wert von 5-9 durchgeführt wird.

9. Verfahren nach einem oder mehreren der Ansprüche 1 und 3-8, dadurch gekennzeichnet, daß die Biotransformation unter Inertgas durchgeführt wird.

10. Verfahren nach Anspruch 1, dadurch gekennzeichnet, daß ein kontinuierliches Verfahren durchgeführt wird oder die Umsetzung "batchweise" erfolgt.

11. Verwendung der nach Anspruch 1 bis 10 hergestellten Verbindungen der allgemeinen Formel I als Bausteine für die Peptidsynthese.

12. Verwendung der nach Anspruch 1 bis 10 hergestellten Verbindungen der allgemeinen Formel I als Bausteine für Bradykinin oder Bradykinin verwandte Peptide.

13. Die Verbindungen der allgemeinen Formel III 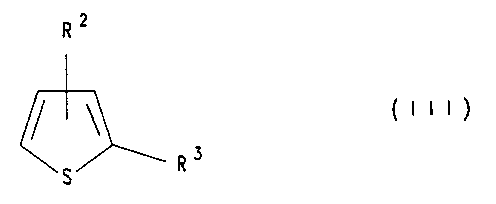 in welcher R² = 4-Brom, 5-Brom, 3-Methyl, 5-Methyl oder 5-Nitro bedeutet und
R³ eine der folgenden Bedeutungen hat

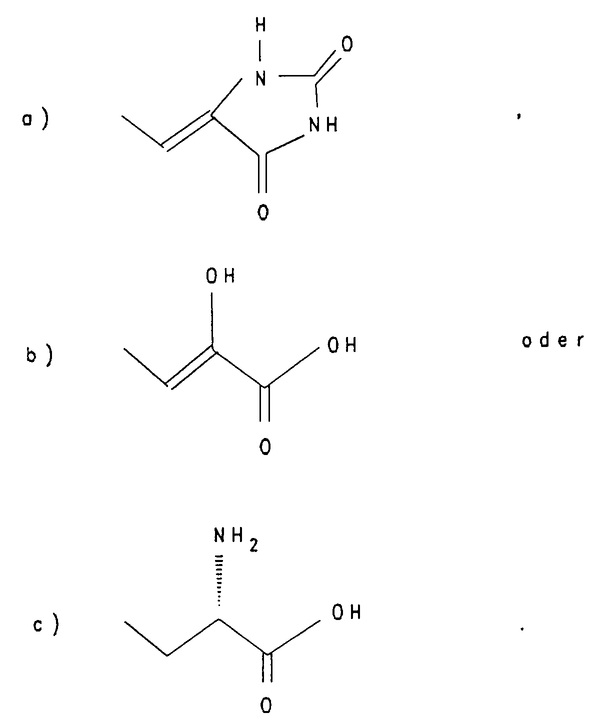

14. Die Verbindungen der allgemeinen Formel IV 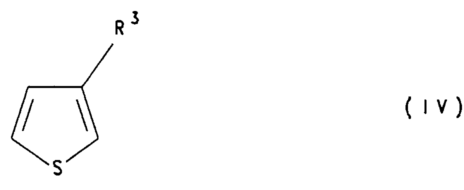 in welcher R³ eine der folgenden Bedeutungen hat:

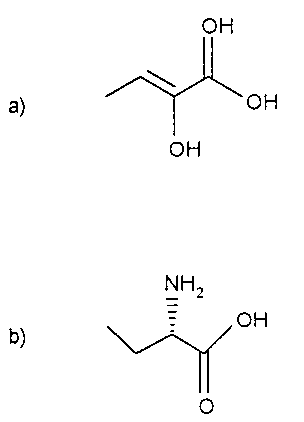

## Claims

1. A process for the preparation of L-thienylalanines of the formula I 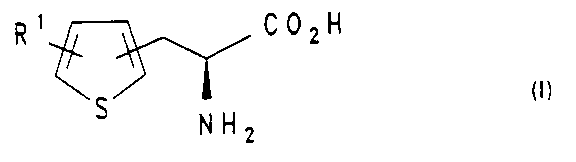 in which R¹ is hydrogen, a halogen radical, a nitro radical or a straight-chain or branched alkyl radical, or the salts of these compounds, which comprises subjecting the compounds of the formula II 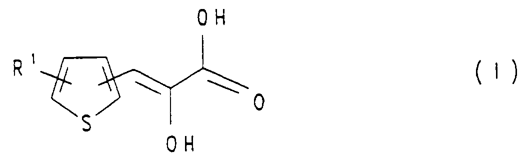 in which R¹ has the abovementioned meanings and the 2-hydroxyacrylic acid radical is in the 2- or 3-position of the thiophene ring, to biotransformation in the presence of the amino acids asparagine, glutamine, L-aspartic acid and/or L-glutamic acid and in the presence of one or more microorganisms and/or enzymes with transaminase activity.

2. The process as claimed in claim 1, wherein the microorganism and/or the enzyme is immobilized on a support.

3. The process as claimed in claim 1, wherein E. coli ATCC 11303 and/or Paracoccus denitrificans DSM 65 are employed.

4. The process as claimed in claim 2, wherein the biotransformation is carried out with E. coli ATCC 11303, transformed with an extrachromosomal element containing the tyrB gene isolated from E. coli ATCC 11303.

5. The process as claimed in claim 1, wherein the biotransformation is carried out using cell extract, isolated complete protein or purified enzyme.

6. The process as claimed in claim 1, wherein the 2-hydroxy-3-thienylacrylic acids, which have been obtained from hydantoin derivatives or from azlactone derivatives, are employed for the biotransformation.

7. The process as claimed in claim 1, wherein amino group donor and the 2-hydroxy-3-thienylacrylic acid in question are employed in a ratio of 1:1 to 5:1.

8. The process as claimed in one or more of claims 1 and 3-7, wherein the biotransformation is carried out at a pH of 5-9.

9. The process as claimed in one or more of claims 1 and 3-8, wherein the biotransformation is carried out under inert gas.

10. The process as claimed in claim 1, wherein the reaction is continuous or batchwise.

11. The use of the compounds of the formula I which have been prepared as claimed in any of claims 1 to 10 as units for peptide synthesis.

12. The use of the compounds of the formula I which have been prepared as claimed in any of claims 1 to 10 as units for bradykinin or bradykinin-related peptides.

13. A compound of the formula III 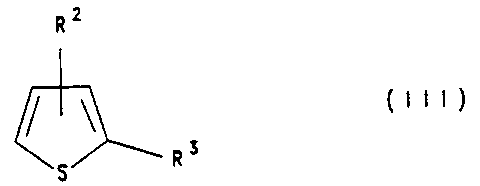 in which R² is 4-bromo, 5-bromo, 3-methyl, 5-methyl or 5-nitro and
R³ has one of the following meanings

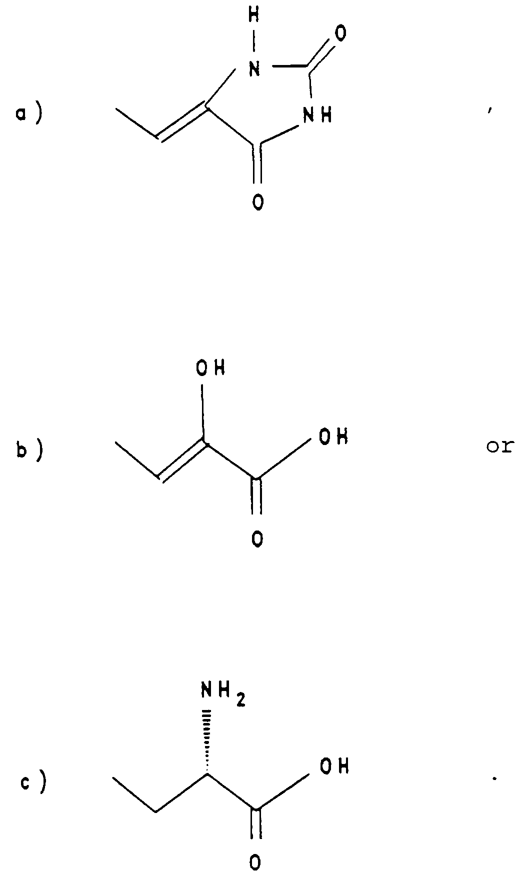

14. A compound of the formula IV 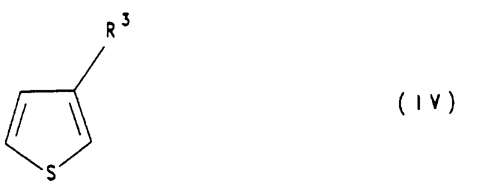 in which R³ has one of the following meanings:

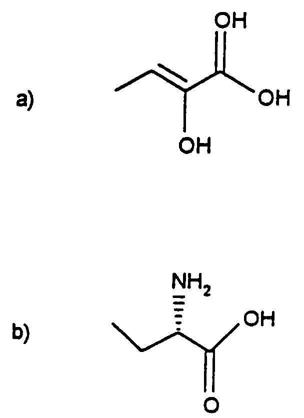

## Revendications

1. Procédé pour la préparation des L-thiénylalanines de formule I 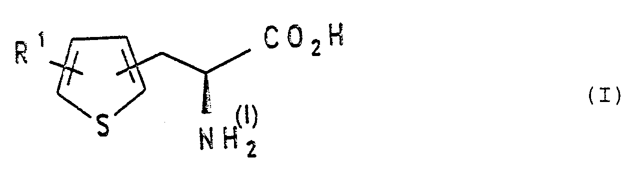 dans laquelle R¹ représente un atome d'hydrogène, un atome d'halogène, un groupe nitro, un reste alkyle linéaire ou ramifié, respectivement les sels de ces composés, caractérisé en ce que l'on réalise la biotransformation des composés de formule II 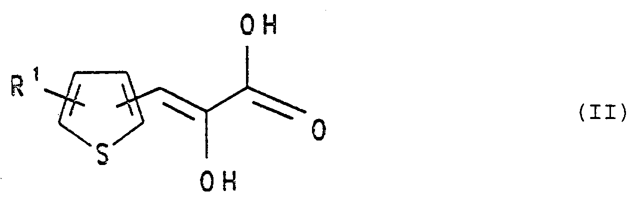 dans laquelle R¹ possède les significations citées ci-dessus et le reste acide 2-hydroxylacrylique en position 2 ou 3 du cycle thiophène, en présence des acides aminés l'asparagine, la glutamine, l'acide L-asparagique et/ou l'acide L-glutamique et en présence de microorganismes ou d'enzymes avec une activité de transaminase.

2. Procédé selon la revendication 1, caractérisé en ce que l'on utilise le microorganisme et/ou l'enzyme immobilisés sur un support.

3. Procédé selon la revendication 1, caractérisé en ce que l'on utilise l'*E. coli* ATCC 11303 et/ou le *Paracoccus denitrificans* DSM 65.

4. Procédé selon la revendication 2, caractérisé en ce que l'on met en oeuvre la biotransformation avec l'*E. coli* ATCC 11303, transformé avec un élément extrachromosomique, qui contient le gène tyrB isolé à partir d'*E. coli* ATCC 11303.

5. Procédé selon la revendication 1, caractérisé en ce que l'on met en oeuvre la biotransformation avec un extrait cellulaire, la protéine totale isolée ou l'enzyme purifiée.

6. Procédé selon la revendication 1, caractérisé en ce que l'on utilise pour la biotransformation les acides 2-hydroxy-3-thiényl-acryliques obtenus à partir des dérivés de l'hydantoïne ou de dérivés de l'azlactone.

7. Procédé selon la revendication 1, caractérisé en ce que l'on utilise le donneur de groupes amino et l'acide 2-hydroxy-3-thiénylacrylique concerné dans un rapport de 1:1 à 5:1.

8. Procédé selon une ou plusieurs des revendications 1 et 3 à 7 caractérisé en ce que l'on met en oeuvre la biotransformation avec une valeur de pH de 5 à 9.

9. Procédé selon une ou plusieurs des revendications 1 et 3 à 8, caractérisé en ce que l'on met en oeuvre la biotransformation sous une atmosphère de gaz inerte.

10. Procédé selon la revendication 1, caractérisé en ce que l'on met en oeuvre un procédé en continu ou l'on réalise la transformation en discontinu.

11. Utilisation des composés de formule générale I préparés selon les revendications 1 à 10 comme éléments pour la synthèse peptidique.

12. Utilisation des composés de formule générale I préparés selon les revendications 1 à 10 comme éléments pour la synthèse de la bradykinine ou de peptides apparentés à la bradykinine.

13. Composés de formule générale III 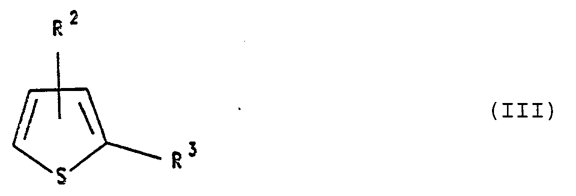 dans laquelle R² représente 4-bromo, 5-bromo, 3-méthyle, 5-méthyle ou 5 nitro
et
R³ possède l'une des significations suivantes

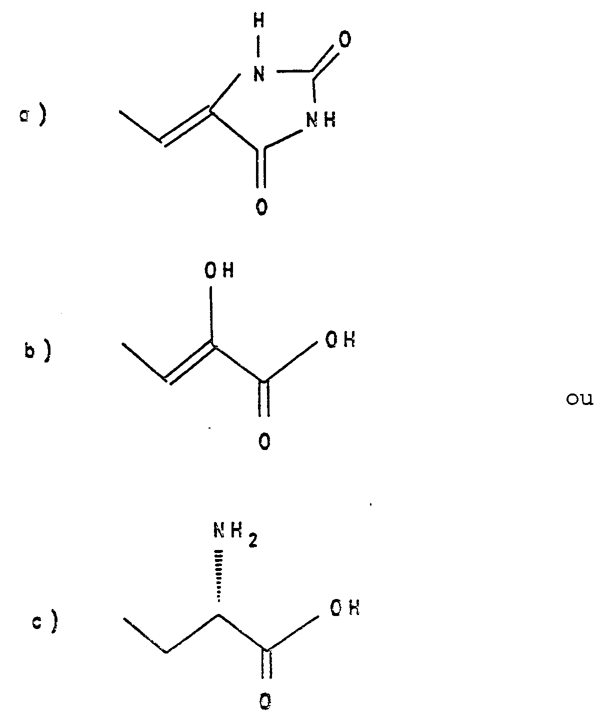

14. Composés de formule générale IV 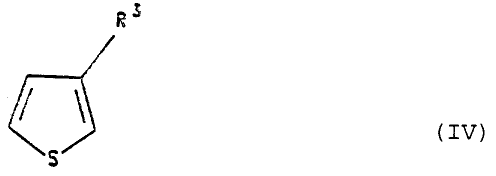 dans laquelle R³ possède l'une des significations suivantes :
